# EUROPÄISCHE PATENTSCHRIFT

(11) **EP 0 374 824 B1**
(45) Veröffentlichungstag und Bekanntmachung des Hinweises auf die Patenterteilung: **22.09.1993**
(21) Anmeldenummer: 89123447.8
(22) Anmeldetag: 19.12.1989
(51) Int. Cl.: A61K 6/083

(54) **Dentalmassen, die bifunktionelle Acrylsäure- oder Methacrylsäureester enthalten**
Dental compositions containing bifunctional acrylic or methacrylic acid esters
Compositions dentaires contenant des esters d'acide acrylique ou méthacrylique bifonctionnels

(30) Priorität: 19.12.1988 DE 3842681
(43) Veröffentlichungstag der Anmeldung: 27.06.1990
(73) Patentinhaber: THERA Patent GmbH & Co. KG Gesellschaft für industrielle Schutzrechte, D-82229 Seefeld (DE)
(72) Erfinder: Jochum, Peter, Dr., D-8031 Seefeld (DE); Guggenberger, Rainer, Dr., D-8031 Hechendorf (DE); Lechner, Günther, Dr., D-8138 Frieding (DE); Ellrich, Klaus, Dr., D-8031 Wörthsee (DE)
(74) Vertreter: Abitz, Walter, Dr.-Ing.

(56) Entgegenhaltungen:
- EP-A- 0 211 104
- EP-A- 0 254 184
- EP-A- 0 284 991
- WO-A-81/01959

## Beschreibung

Die Erfindung betrifft Dentalmassen, die
(a) 10 - 99,999 Gew.-% eines mindestens bifunktionellen Acrylsäure- und/oder Methacrylsäureesters,
(b) 0,001 bis 5 Gew.-% eines Initiatorsystems, welches die radikalische Polymerisation auslösen kann, und
(c) 0 bis 89,999 Gew.-% Füllstoffe, Thixotropie-Hilfsmittel und andere Hilfsstoffe,
bezogen auf die Gesamtmasse von (a) + (b) + (c), enthalten.

Polymerisierbare Dentalmassen, beispielsweise Zahnfüllmassen, provisorische Kronen- und Brückenmaterialien oder Zementierungsmaterialien, bestehen aus Kunststoffen, die mit organischen oder anorganischen Füllkörpern versehen sind. Je nach Anwendungszweck hat man es hierbei mit dünnfliessenden bis zähplastischen Massen zu tun. Provisorische Kronen- und Brückenmaterialien z.B. müssen zum einen ein gutes Fliessverhalten zeigen, um in die genommene Abformung überall hineinzufliessen, zum anderen dürfen sie nicht zu fliessfähig sein, um beim Anpassen im Kiefer nicht wieder aus der Abformung herauszufliessen. Solche provisorischen Kronen- und Brückenmaterialien sind üblicherweise relativ niedrig gefüllte Systeme, die ca. 10 bis 70 Gew.-% anorganisches Füllmaterial enthalten. Die verwendeten Füllstoffe haben üblicherweise eine mittlere Korngrösse von 1 bis 15 µm. Zusätzlich werden aber auch wesentlich feinere Füllstoffe im Bereich von 0,02 bis 0,05 µm mit den oben genannten Füllstoffen eingesetzt, um die Massen ausreichend plastisch und thixotrop zu machen. Bei provisorischen Kronen- und Brückenmaterialien hat sich auch die Verwendung von organischen Füllstoffen, wie Polymethylmethacrylat, bewährt.

Bei der Herstellung solcher Dentalmassen war man in der Vergangenheit immer bemüht, dem Zahnarzt zwar eine ausreichend lange Verarbeitungszeit zur Verfügung zu stellen, aber die Abbindephase (das ist der Zeitabschnitt vom Gelierungsbeginn bis zu dem Zeitpunkt, an dem die Härtung der Masse beendet ist) und damit die Behandlungszeit des Patienten so kurz wie möglich zu halten. Hierzu war es nötig, möglichst reaktive Systeme, wie Photoinitiatoren oder Redoxkatalysatoren, die die radikalische Polymerisation von Acrylsäure- und Methacrylsäureestern in kurzer Zeit bewirken, einzusetzen. So beschreibt beispielsweise die europäische Patentanmeldung 0 059 451 die Verwendung bestimmter Malonylsulfamide als Aktivator für die peroxidische Polymerisation von polymerisierbaren Dentalmassen. Einer der dort genannten Vorteile ist die kurze Abbindephase trotz langer Verarbeitungszeit. In ähnlicher Weise beschreibt die deutsche Offenlegungsschrift 14 95 520 ein Verfahren zum Polymerisieren von Acrylsäureestern, bei dem ein Katalysatorsystem, bestehend aus organischem Peroxid, ionogenem Halogen, einer Kupferbindung und einer Barbitursäure als Aktivator verwendet wird.

Auch bei der Verwendung von Photoinitiatoren war man bestrebt, möglichst reaktive Systeme, die eine schnelle Polymerisation in der kurzen Bestrahlungsphase ermöglichen, für polymerisierbare Dentalmassen einzusetzen. So beschreibt beispielsweise die europäische Patentveröffentlichungsschrift 1 84 095 besonders reaktive Bisacylphosphinoxide als Photoinitiatoren für Dentalmassen.

Der Einsatz möglichst reaktiver Initiatorsysteme für die Polymerisation von Dentalmassen auf Acrylsäure- oder Methacrylsäureesterbasis hat zwar zu dem Vorteil geführt, dass man in sehr kurzen Zeiten harte Kunststoffmaterialien erhalten kann; für den Zahnarzt ergab sich aber durch die extrem schnelle Abbindephase das Problem, dass bei einigen Anwendungszwecken die Zeit zum Ausarbeiten und Verarbeiten von Dentalmaterialien, insbesondere zum Entnehmen von provisorischen Kronen- und Brückenmaterialien, zu kurz wurde.

Aus der europäischen Patentanmeldung 211 104 sind Klebstoffmassen bekannt, die Vinylverbindungen und Verbindung der allgemeinen Formel 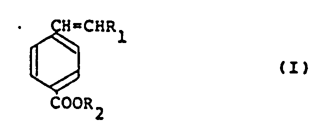
enthalten, worin R₁ Wasserstoff oder Methyl und R₂ Wasserstoff oder einen gegebenenfalls substituierten Alkylrest mit 1 bis 5 Kohlenstoffatomen bedeuten. Als Vinylverbindungen werden unter anderem auch bifunktionelle Methacrylsäureester erwähnt. Weiterhin sind aus der deutschen Offenlegungsschrift 31 20 965 gehärtete Harze und deren Verwendung zur Herstellung von optischen Linsen bekannt, die aus bifunktionellen Acrylsäure- oder Methacrylsäureestern sowie einer weiteren ungesättigten Verbindung hergestellt worden ist, wobei die weitere ungesättigte Verbindung Styrol, α-(C₁₋₃)-Alkylstyrol, Vinylnaphthalin, etc. sein kann. Versuche haben jedoch ergeben, dass ungesättigte Verbindung mit nur einem aromatischen Rest, wie sie in den beiden genannten Veröffentlichung beschrieben werden, keine zufriedenstellende Wirksamkeit hinsichtlich der Verlängerung der Abbindephase entfalten. Derartige Zusätze wirken allenfalls nur in sehr hohen Konzentrationen und auch dann nicht in einem zufriedenstellenden Masse. So konnte festgestellt werden, dass das in der erwähnten Offenlegungsschrift beispielsweise erwähnte α-Methylstyrol praktisch keine Verlängerung der Abbindephase bewirken kann. Darüber hinaus weisen diese Art von Verbindungen aufgrund ihrer niedrigen Flüchtigkeit Probleme bei der Lagerhaltung und Verarbeitung auf, und für viele Anwendungsbereiche, z.B. in Dentalmassen, ist ein Zusatz von leicht flüchtigen Verbindungen unerwünscht.

Aufgabe der Erfindung ist daher die Bereitstellung von polymerisierbaren Dentalmassen, insbesondere provisorischen Kronen- und Brückenmaterialien, deren Abbindephase verlängert ist, so dass sie länger verarbeitbar sind, und die dennoch zu einer ausgezeichneten Endhärte des polymerisierten Dentalmaterials führen.

Gegenstand der Erfindung sind Dentalmassen, die
(a) 10 - 99,999 Gew.-% eines mindestens bifunktionellen Acrylsäure- und/oder Methacrylsäureesters,
(b) 0,001 bis 5 Gew.-% eines Initiatorsystems, welches die radikalische Polymerisation auslösen kann, und
(c) 0 bis 89,999 Gew.-% Füllstoffe, Thixotropie-Hilfsmittel und andere Hilfsstoffe,
bezogen auf die Gesamtmasse von (a) + (b) + (c), enthalten, und die dadurch gekennzeichnet sind, dass sie ausserdem
(d) 0,001 bis 10 Gew.-%, bezogen auf (a) einer Verbindung der allgemeinen Formel 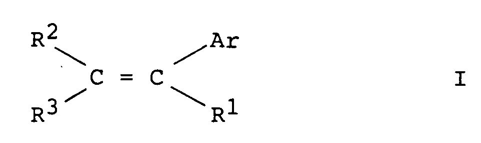 enthalten, in der bedeuten:
   Ar Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist,
   R¹, R² und R³, die gleich oder verschieden sind, Wasserstoff, Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, geradkettiges oder verzweigtes C₁₋₁₈-Alkyl, C₁₋₁₈-Alkoxyl oder C₁₋₁₇-Alkoxycarbonyl, wobei Alkyl und Alkoxyl durch Halogen oder Aryl substituiert sein können,
   wobei, wenn R¹ oder R² Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, C₁₋₁₈-Alkyl oder C₁₋₁₈-Alkoxyl be deutet, dieser Rest mit Ar durch eine Einfachbindung verknüpft sein kann und
   wobei, wenn Ar Phenyl, C₁₋₁₈-Alkylphenyl, C₁₋₁₈-Alkoxyl phenyl, Carboxyl-C₁₋₁₇-alkylphenyl oder Halogenphenyl bedeutet, R² auch -O- bedeuten kann, das mit dem Phenylrest von Ar zu einem Benzofuran verknüpft ist, und
   wobei mindestens einer der Reste R¹ bis R³ H und mindestens einer der Reste R¹ bis R³ Aryl oder durch geradkettiges oder verzweigtes C₁₋₁₈-Alkyl, C₁₋₁₈-Alkoxyl oder Carboxyl-C₁₋₁₇-alkyl oder Halogen substituiertes Aryl bedeuten.

Bevorzugte Dentalmassen enthalten als (d) Verbindungen der allgemeinen Formel I, in der Ar Phenyl, Naphthyl oder Anthryl bedeutet, die durch C₁₋₁₈-Alkyl, C₁₋₁₈-Alkoxyl, Carboxyl-C₁₋₁₇-alkyl oder Halogen substituiert sein können.

Vorzugsweise haben zwei der Reste R¹ bis R³ der Formel I die Bedeutung H, vorzugsweise sind das die Reste R² und R³. Weiter bevorzugt sind Verbindungen der allgemeinen Formel I, bei denen R¹ und Ar gleich sind und die Bedeutung Phenyl, C₁-C₆-Alkylphenyl, C₁-C₆-Alkoxylphenyl oder Carboxyl-C₁₋₆-alkylphenyl haben. Bevorzugt sind auch Verbindungen der allgemeinen Formel I, bei denen R¹ die Bedeutung von Ar hat, R¹ und Ar aber voneinander verschieden sind.

Vorzugsweise enthalten die Dentalmassen den Bestandteil (d) in einer Menge von 0,01 bis 5 Gew.-%, ganz besonders bevorzugt 0,05 bis 2 Gew.-%, bezogen auf den Bestandteil (a).

Ein Grossteil der Verbindungen der allgemeinen Formel I ist handelsüblich und/oder ihr Herstellungsverfahren ist bekannt. So lässt sich beispielsweise 1,1-Diphenylethylen durch Umsetzung von Phenylmagnesiumbromid mit Essigsäureester und anschliessende Wasserabspaltung herstellen, siehe z.B. Organic Synthesis Coll. Vol. I, Seite 226 ff. (1948, 2. Ausgabe).

Verbindungen der Formel I, in denen die Reste R² und R³ gleich H sind, lassen sich auch in bekannter Weise aus den entsprechenden Carbonylverbindungen über die sog. Wittig-Synthese erhalten, siehe z.B. Macro Molecules 9, 716 (1976).

Die Erfindung betrifft auch die Verwendung einer Verbindung der allgemeinen Formel I bei der Herstellung von Dentalmassen, die bifunktionelle Acrylsäure- und/oder Methacrylsäureester enthalten.

Die erfindungsgemäss als Bestandteil (a) einzusetzenden, mindestens bifunktionellen Acrylsäure- und/oder Methacrylsäureester können monomere und polymere Acrylate und Methacrylate beinhalten. Vorteilhaft verwendet werden können beispielsweise die langkettigen Monomeren der US-PS 3 066 112 auf der Basis von Bisphenol A und Glycidylmethacrylat oder deren durch Addition von Isocyanaten entstandene Derivate. Geeignet sind auch Verbindungen des Typs Bisphenol-A-diethyloxy(meth)-acrylat und Bisphenol-A-dipropyloxy(meth)-acrylat. Weiterhin Verwendung finden können die oligo-ethoxylierten und oligo-propoxylierten Bisphenol-A-diacryl-und -dimethacrylsäureester.

Gut geeignet sind weiter die Acrylsäure- und Methacrylsäureester mindestens bifunktioneller aliphatischer Alkohole, beispielsweise Triethylenglykol-di(meth)-acrylat, Ethylenglykol-di(meth)-acrylat, Hexandiol-di(meth)-acrylat und Trimethylolpropan-tri(meth)-acrylat.

Besonders geeignet sind auch die in der deutschen Patentschrift 28 16 823 genannten Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und/oder Propylenoxideinheiten verlängerten Verbindungen des Bis-(hydroxymethyl)-tricyclo[5.2.1.0^{2,6}]-decans.

Gut geeignete Monomere sind auch die in der europäischen Veröffentlichungsschrift 0235826 beschriebenen Methacrylsäureester, z.B. Triglykolsäure-bis[3(4)-methacryloxymethyl-8(9)-tricyclo[5.2.1.0^{2,6}]-decylmethylester].

Selbstverständlich können auch Gemische aus Monomeren und/oder aus hieraus hergestellten ungesättigten Polymeren verwendet werden.

Zusätzlich zu den mindestens bifunktionellen Acrylsäure-und Methacrylsäureestern können bis 70 Gew.-%, bezogen auf Bestandteil (a), vorzugsweise bis 50 Gew.-% monofunktionelle Methacrylsäureester, wie Methylmethacrylat, eingesetzt werden.

Als Bestandteil (b) eignen sich Initiatorsysteme, die die radikalische Polymerisation der mindestens bifunktionellen Monomeren bewirken, z.B. Photoinitiatoren oder sogenannte Redoxinitiatorsysteme.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyl-diphenyl-phosphinoxid und Bis-(2,6-dichlorbenzoyl)-4-n-propylphenyl-phosphinoxid. Es eignen sich aber auch andere Verbindungen dieses Typs, wie sie in den europäischen Patentveröffentlichungsschriften 73 413, 7 508, 47 902, 57 474 und 184 095 beschrieben sind.

Die Konzentration der Photoinitiatoren beträgt vorzugsweise 0,01 bis 3 Gew.-%, besonders bevorzugt 0,1 bis 2 Gew.-%, bezogen auf die Gesamtmasse von (a) + (b) + (c).

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid in Betracht.

Als Aktivatoren eignen sich beispielsweise tertiäre aromatische Amine, wie die aus der US-PS 35 41 068 bekannten N,N-Bis-(hydroxyalkyl)-3,5-xylidine sowie die aus der deutschen Offenlegungsschrift 26 58 530 bekannten N,N-Bis-(hydroxyalkyl)-3,5-di-t-butylaniline, insbesondere N,N-Bis-(β-oxybutyl)-3,5-di-t-butylanilin.

Gut geeignete Aktivatoren sind auch die in der deutschen Auslegeschrift 14 95 520 beschriebenen Barbitursäuren und Barbitursäurederivate sowie die in der europäischen Patentschrift 0 059 451 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethyl-malonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt, beispielsweise genannt seien die löslichen Aminhydrochloride sowie quartäre Ammoniumchloridverbindungen.

Wenn die erfindungsgemässen Dentalmassen als (b) ein Redoxinitiatorsystem aus organischem Peroxid und Aktivator enthalten, so sind vorzugsweise Peroxid und Aktivator in räumlich voneinander getrennten Teilen der erfindungsgemässen Dentallmasse vorhanden, die erst unmittelbar vor der Anwendung der erfindungsgemässen Dentalmasse homogen miteinander vermischt werden. Enthält die erfindungsgemässe Dentalmasse als (b) nebeneinander organisches Peroxid, Kupferverbindung, Halogenid und Malonylsulfamid, so ist es insbesondere sinnvoll, dass organisches Peroxid, Malonylsulfamid und die Kombination Kupferverbindung/Halogenid in drei räumlich voneinander getrennten Bestandteilen vorliegen. Beispielsweise können organisches Peroxid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise einem Phthalat, zu zwei separaten Pasten verknetet sein. Andererseits können die polymerisierbaren Monomeren auch zusammen mit Kupferverbindung/Halogenid und Füllern vorliegen. Der Bestandteil (d) kann, falls die erfindungsgemässe Dentalmasse in räumlich voneinander getrennten Bestandteilen vorliegt, in jedem dieser Bestandteile vorhanden sein.

Ausser den mindestens bifunktionellen Acrylsäure- und Methacrylsäureestern (a) und dem Initiatorsystem (b) können bis zu 89,999 Gew.-%, bezogen auf die Gesamtmasse von (a) + (b) + (c), organische und/oder anorganische Füllstoffe, Pigmente, Farbstoffe, Thixotropie-Hilfsmittel, Weichmacher und andere Hilfsstoffe enthalten sein.

Anorganische Füllstoffe können beispielsweise Quarz, gemahlene Gläser, schwer lösliche Fluoride, wie CaF₂, YF₃, Kieselgele sowie Kieselsäure, insbesondere pyrogene Kieselsäure oder deren Granulate, sein. Sie sind vorzugsweise in einer Konzentration von 2 bis 85 Gew.-%, ganz besonders bevorzugt von 30 bis 70 Gew.-%, bezogen auf die Gesamtmasse von (a) + (b) + (c), in den Dentalmassen enthalten. Zum besseren Einbau in die Polymermatrix kann es von Vorteil sein, die Füllstoffe sowie gegebenenfalls röntgenopake Zusatzstoffe, wie Yttriumfluorid, zu hydrophobieren. Übliche Hydrophobierungsmittel sind Silane, beispielsweise Trimethoxymethacryoxypropylsilan. Die maximale mittlere Korngrösse der anorganischen Füllstoffe beträgt vorzugsweise 15 µm, insbesondere 10 µm. Ganz besonders bevorzugt werden Füllstoffe mit einer mittleren Korngrösse von < 5 µm eingesetzt.

Als Füllstoffe eignen sich auch bereits fertig pigmentierte Polymethylmethacrylatperlen oder andere pulverisierte organische Polymerisate. Zur Erhöhung der Flexibilität der Massen kann es auch vorteilhaft sein, lösliche organische Polymere einzusetzen. Geeignet sind z.B. Polyvinylacetat sowie die Copolymeren auf Basis Vinylchlorid/Vinylacetet, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate.

Die erfindungsgemässen Dentalmassen haben folgende Vorteile:
1. Bei erfindungsgemäss photopolymerisierbaren Dentalmassen wird durch den Einsatz des Bestandteils (d) erreicht, dass die Polymerisation nicht mehr schlagartig innerhalb weniger Sekunden (10-20 sec) zu harten Polymeren führt, sondern die Polymerisation über einen Zeitraum von 20 bis 60 Sekunden gleichmässig vonstatten geht und somit Spannungen innerhalb des Polymeren vermieden werden können. Wird die erfindungsgemässe Dentalmasse nur kürzer belichtet (10-30 sec), so ist das Material mechanisch noch gut bearbeitbar und kann in nachfolgenden Belichtungsschritten nachgehärtet werden. Insbesondere bei Zahnfüllmassen ergeben sich somit bessere Ergebnisse bei Legen von Zahnfüllungen bezüglich Randspalt und Haftung am umgebenden Zahnmaterial. Auch das Auftreten von Mikrorissen infolge von Spannungen wird vermindert, wodurch die Abrasionsbeständigkeit des Materials erhöht wird. Ein weiterer Vorteil ist es, dass durch die langsame Abbindung ein nur mässiger Temperaturanstieg infolge der frei werdenden Reaktionswärme bei der Polymerisation auftritt.
2. Bei erfindungsgemäss redoxpolymerisierten Dentalmassen erhält man zusätzlich zu oben genannten Vorteilen noch eine gute Bearbeitbarkeit der Massen nach eingesetzter Polymerisation, d.h. dass der gummielastische Übergangszustand länger erhalten bleibt und somit Überschüsse beim Zementieren und Legen einer Füllung noch gut entfernbar sind.
3. Einen besonderen Vorteil bieten die erfindungsgemässen Massen bei der Verwendung als provisorisches Kronen- und Brückenmaterial. Durch die längere gummielastische Abbindephase ist es für den Zahnarzt möglich, das Provisorium in noch nicht völlig ausgehärtetem Zustand aus dem Mund des Patienten zu entnehmen und weiter zu bearbeiten, ohne dass hierbei die Formbeständigkeit des Provisoriums gefährdet ist. Gegenüber bisherigen Materialien steht zur Entnahme des Provisoriums ein Zeitbereich von mehreren Minuten zur Verfügung, so dass auch Temperatureinflüsse, Grösse des Provisoriums und Lagerung der Materialien die Verarbeitbarkeit des Provisoriums kaum beeinflussen.

### Beispiel 1: Polymerisierbare Dentalmasse

100 g 2,2-Bis{4-[oligo(ethoxy)]-phenyl}-propan-dimethacrylat (Diacryl-121, Fa. Akzo), 0,3 g (β-Phenylethyl)-dibutyl-ammonium-chlorid, 60 mg Bis-(1-phenylpentan-1,3-dionato)-kupfer (II) sowie die den Angaben in Tabelle 1 entsprechenden Mengen an erfindungsgemäss verwendeter Verbindung (d) werden zu einer homogenen Lösung vermischt. Die so hergestellte Lösung wird mit 21 g zahnähnlich eingefärbter, silanisierter, mikrofeiner Kieselsäure zu einer noch fliessfähigen Paste verknetet. 75 g dieser Paste werden mit 3 g einer Lauroylperoxidpaste (25%ig in Dioctylphthalat) und 3 g einer Paste, enthaltend 25 Gew.-% 2,6-Dibutyl-4-isobutylmalonylsulfamid in Dioctylphthalat, homogen vermischt. Aus dieser Mischung werden die in der Tabelle genannten Abbindephasen mittels eines Curometers (Fa. Wallace-Shawberry) bestimmt.

**Tabelle 1**

| Nr. | Verbindung (d) | zugesetzte Menge an (d) in Gew.-% bezogen auf (a) | Zeitablesung für Abbindephase im Curometer bei 23°C | Abbindephase (Min.) |
|---|---|---|---|---|
| 1 | - | 0 | 3'30" bis 6'50" | 3'20" |
| 2 | 2,5-Dimethylstyrol | 2 | 3'30" bis 7'10" | 3'10" |
| 3 | Trans-Stilben | 2 | 5'30" bis 11'30" | 6'00" |
| 4 | 1,2-Dihydro-4-phenylnaphthalin | 2 | 3'50" bis 9'00" | 5'10" |

### Beispiel 2

Aus 100 g Diacryl-121 (siehe Beispiel 1), 0,3 g N,N-Dibutyl-β-phenylethylaminhydrochlorid und jeweils 0,06 g Verbindung (d) wird eine homogene Lösung hergestellt. Zu dieser Lösung werden 21 g zahnähnlich eingefärbte, silanisierte, mikrofeine Kieselsäure gegeben und zu einer noch fliessfähigen Paste verknetet. Anschliessend werden 75 g dieser Paste mit je 3 g der in Beispiel 1 beschriebenen Lauroylperoxidpaste und Malonylsulfamidpaste homogen vermischt und mittels eines Curometers die Abbindephase bestimmt.

**Tabelle 2**

| Nr. | Verbindung (d) | Zeitablesung für Abbindephase im Curometer bei 23°C | Abbindephase (Min.) |
|---|---|---|---|
| 5 | - | 1'10" bis 3'00" | 1'50" |
| 6 | α-Methylstyrol | 1'10" bis 3'05" | 1'55" |
| 7 | 1,1-Diphenylethylen | 2'30" bis 13'00" | 10'30" |
| 8 | 1,1-Bis(-4-Methoxyphenyl)ethylen | 3'40" bis 12'00" | 8'20" |
| 9 | 1-(4-Methoxyphenyl)-1-phenylethylen | 2'50" bis 12'00" | 9'10" |
| 10 | 1-(3,4-Dimethylphenyl)1-phenylethylen | 2'40" bis 11'30" | 8'50" |
| 11 | 1-(4-Chlorphenyl)-1-phenylethylen | 2'25" bis 11'40" | 9'15" |
| 12 | 1-(2,5-Dimethylphenyl)-1-phenyl ethylen | 1'40" bis 5'30" | 3'50" |

### Beispiel 3: Anwendungsbeispiel, Herstellung einer semipermanenten Brücke

1,5 g der aktivierten Mischung nach-Beispiel 2, Nr. 7, werden mit einer Spritze in einen Alginatabdruck eingebracht, der vor der Präparation der Brückenpfeilerzähne im Patientenmund gewonnen wurde und in dem eine tiefe Rille zwischen den Abdrükken der Pfeilerzähne eingeschnitten ist. Der gefüllte Abdruck wird in den Mund des Patienten eingesetzt und nach Beginn der Abbindephase (innerhalb 4 bis 10 Minuten nach Mischbeginn) mit dem Formkörper wieder aus dem Mund entfernt. Während der Abbindephase tritt nur eine geringe Temperaturerhöhung auf. Tabelle 3 zeigt einige physikalische Werte. Die Druckfestigkeit des erhärteten Materials wurde anhand von zylindrischen Prüfkörpern mit einem Durchmesser von 4 mm und einer Höhe von 8 mm bestimmt, die Biegefestigkeit anhand von Prüfkörpern der Abmessungen 4 x 4 x 25 mm.

**Tabelle 3**

| Material | Entnahmezeitraum | erhärtetes Material | |
|---|---|---|---|
| | | Druckfestigkeit | Biegefestigkeit |
| Beispiel 2, Nr. 7 (erfindungsgemäss) | 5 - 10 Min. nach Anmischbeginn | 270 MPa | 90 MPa |
| "Protemp", Fa. ESPE (Vergleich) | 5 - 6 Min. nach Anmischbeginn | 180 MPa | 80 MPa |
| Acrylatbasis, "Trim", Fa. Bosworth (Vergleich) | 4 - 8 Min. nach Anmischbeginn | 70 MPa | 60 MPa |

### Ergebnis

Verglichen mit einem handelsüblichen Material auf Basis Dimethacrylsäureester (Protemp, Fa. Espe) hat das erfindungsgemässe Material deutlich höhere mechanische Festigkeiten aufgrund der verringerten Spannungen bei der Polymerisation und einen erheblich verlängerten Entnahmezeitraum, was die Verarbeitung beim Zahnarzt deutlich erleichtert. Gegenüber einem klassischen Material auf Basis monofunktioneller Acrylate (Trim, Fa. Bosworth) hat das erfindungsgemässe Material ebenfalls einen erhöhten Entnahmezeitraum und deutlich bessere mechanische Eigenschaften.

### Beispiel 4: Photopolymerisierbare Lösung

98,8 Gew.-% Bis-(acryloyloxymethyl)-tricyclo[5.2.1.0^{2,6}]-decan, 1 Gew.-% Dimethylethanolamin und 0,2 Gew.-% Campherchinon werden miteinander so lange verrührt, bis eine homogene, klare gelbliche Lösung entsteht (Herstellung der Mischung unter Ausschluss von Tageslicht). Zu den Lösungen werden die in der Tabelle angegebenen Gew.-% an Diphenylethylen hinzugegeben und die Lösungen anschliessend so lange mit einem dentalen Halogenlichtgerät (ELIPAR, Fa. ESPE, 400 bis 500 nm) bestrahlt, bis die maximale Reaktionstemperatur erreicht ist (Messung in einer zylindrischen Form, ø 3 mm, Höhe: 2 mm).

**Tabelle 4**

| Nr. | Gew.-% Diphenylethylen, bezogen auf (a) | Temperaturmaximum (°C) | Zeit zur Erreichung des Temperaturmaximums (sec.) |
|---|---|---|---|
| 13 | 0 | 80 | 18 |
| 14 | 0,03 | 78 | 24 |
| 15 | 0,06 | 74 | 25 |

### Ergebnis

Verglichen mit einem Material ohne den erfindungsgemäss verwendeten Zusatz zeigen die erfindungsgemässen Zusammensetzungen eine deutlich verlängerte Abbindephase, die zudem noch zu geringeren Temperaturspitzen bei der Polymerisation führt.

## Patentansprüche

1. Dentalmassen, die
(a) 10 - 99,999 Gew.-% eines mindestens bifunktionellen Acrylsäure- und/oder Methacrylsäureesters,
(b) 0,001 bis 5 Gew.-% eines Initiatorsystems, welches die radikalische Polymerisation auslösen kann, und
(c) 0 bis 89,999 Gew.-% Füllstoffe, Thixotropie-Hilfsmittel und andere Hilfsstoffe,
bezogen auf die Gesamtmasse von (a) + (b) + (c), enthalten, dadurch gekennzeichnet, dass sie ausserdem
(d) 0,001 bis 10 Gew.-%, bezogen auf (a), einer Verbindung der allgemeinen Formel 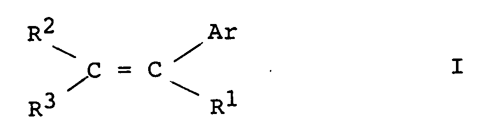 enthalten, in der bedeuten:
Ar Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist,
R¹, R² und R³, die gleich oder verschieden sind, Wasserstoff, Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, geradkettiges oder verzweigtes C₁₋₁₈-Alkyl, C₁₋₁₈-Alkoxyl oder C₁₋₁₇-Alkoxycarbonyl, wobei Alkyl und Alkoxyl durch Halogen oder Aryl substituiert sein können,
wobei, wenn R¹ oder R² Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, C₁₋₁₈-Alkyl oder C₁₋₁₈-Alkoxyl bedeutet, dieser Rest mit Ar durch eine Einfachbindung verknüpft sein kann und
wobei, wenn Ar Phenyl, C₁₋₁₈-Alkylphenyl, C₁₋₁₈-Alkoxylphenyl, Carboxyl-C₁₋₁₇-alkylphenyl oder Halogenphenyl bedeutet, R² auch -O- bedeuten kann, das mit dem Phenylrest von Ar zu einem Benzofuran verknüpft ist, und
wobei mindestens einer der Reste R¹ bis R³ H und mindestens einer der Reste R¹ bis R³ Aryl oder durch geradkettiges oder verzweigtes C₁₋₁₈-Alkyl, C₁₋₁₈Alkoxyl oder Carboxyl-C₁₋₁₇-alkyl oder Halogen substituiertes Aryl bedeuten.

2. Dentalmassen nach Anspruch 1, dadurch gekennzeichnet, dass sie als (d) Verbindungen der allgemeinen Formel I enthalten, in der Ar Phenyl, Naphthyl oder Anthryl bedeutet, die durch C₁₋₁₈-Alkyl, C₁₋₁₈-Alkoxyl, Carboxyl-C₁₋₁₇-alkyl oder Halogen substituiert sein können.

3. Dentalmassen nach Anspruch 1 oder 2, dadurch gekennzeichnet, dass sie als (d) Verbindungen der allgemeinen Formel I enthalten, in der mindestens zwei der Reste R¹, R², R³, besonders R² und R³ Wasserstoff bedeuten.

4. Dentalmassen nach Anspruch 3, dadurch gekennzeichnet, dass sie als (d) Verbindungen der allgemeinen Formel I enthalten, in denen R¹ und Ar gleich sind und Phenyl, C₁₋₆-Alkylphenyl, C₁₋₆-Alkoxylphenyl oder Carboxyl-C₁₋₆-alkylphenyl bedeuten.

5. Verwendung von Verbindungen der allgemeinen Formel 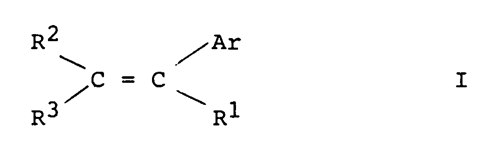 in der bedeuten:
Ar Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist,
R¹, R² und R³, die gleich oder verschieden sind, Wasserstoff, Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, geradkettiges oder verzweigtes C₁₋₁₈-Alkyl, C₁₋₁₈-Alkoxyl oder C₁₋₁₇-Alkoxycarbonyl, wobei Alkyl und Alkoxyl durch Halogen oder Aryl substituiert sein können,
wobei, wenn R¹ oder R² Aryl oder substituiertes Aryl, welches durch geradkettiges oder verzweigtes C₁₋₁₈-Alkyl, C₁₋₁₈-Alkoxyl oder Carboxyl-C₁₋₁₇-alkyl oder Halogen substituiert ist, C₁₋₁₈-Alkyl oder C₁₋₁₈-Alkoxyl bedeutet, dieser Rest mit Ar durch eine Einfachbindung verknüpft sein kann und
wobei, wenn Ar Phenyl, C₁₋₁₈-Alkylphenyl, C₁₋₁₈-Alkoxylphenyl, Carboxyl-C₁₋₁₇-alkylphenyl oder Halogenphenyl bedeutet, R² auch -O- bedeuten kann, das mit dem Phenylrest von Ar zu einem Benzofuran verknüpft ist, und
wobei mindestens einer der Reste R¹ bis R³ H und mindestens einer der Reste R¹ bis R³ Aryl oder durch geradkettiges oder verzweigtes C₁₋₁₈-Alkyl, C₁₋₁₈-Alkoxyl oder Carboxyl-C₁₋₁₇-alkyl oder Halogen substituiertes Aryl bedeuten.
bei der Herstellung von Dentalmassen, die bifunktionelle Acrylsäure- und/oder Methacrylsäureester enthalten.

## Claims

1. Dental compounds which contain, based on the total weight of (a) + (b) + (c),
(a) 10 - 99.999 % by weight of an at least bifunctional acrylic-acid and/or methacrylic-acid ester,
(b) 0.001 to 5 % by weight of an initiator system which can initiate the radical polymerization, and
(c) 0 to 89.999 % by weight of fillers, thixotropic agents and other auxiliary agents,
characterised in that in addition they contain
(d) 0.001 to 10 % by weight, referred to (a), of a conpound of the general formula 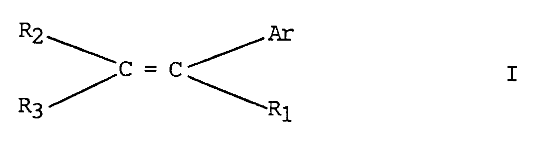 in which:
Ar represents aryl or substituted aryl, which is substituted by straight-chain or branched C₁₋₁₈-alkyl, C₁₋₁₈-alkoxyl or carboxyl-C₁₋₁₇-alkyl or halogen,
R¹, R² and R³, which are the same or different, represent hydrogen, aryl or substituted aryl, which is substituted by straight-chain or branched C₁₋₁₈-alkyl, C₁₋₁₈-alkoxyl or carboxyl-C₁₋₁₇-alkyl or halogen, or represent straight-chain or branched C₁₋₁₈-alkyl, C₁₋₁₈-alkoxyl or C₁₋₁₇-alkoxycarbonyl, in which alkyl and alkoxyl can be substituted by halogen or aryl,
in which, if R¹ or R² represents aryl or substituted aryl, which is substituted by straight-chain or branched C₁₋₁₈-alkyl, C₁₋₁₈-alkoxyl or carboxyl-C₁₋₁₇₋alkyl or halogen, or represents C₁₋₁₈-alkyl or C₁₋₁₈-alkoxyl, this radical can be linked with Ar by a single bond and
in which, if Ar represents phenyl, C₁₋₁₈-alkylphenyl, C₁₋₁₈-alkoxylphenyl, carboxyl-C₁₋₁₇-alkylphenyl or halogenphenyl, R² can also represent -O-, which is linked with the phenyl radical of Ar to a benzofuran,
and
in which, at least one of the radicals R¹ to R³ represents H and at least one of the radicals R¹ to R³ represents aryl or aryl substituted by straight-chain or branched C₁₋₁₈-alkyl, C₁₋₁₈-alkoxyl or carboxyl-C₁₋₁₇-alkyl or halogen.

2. Dental compounds according to claim 1, characterised in that as (d) they contain compounds of the general formula I in which Ar represents phenyl, naphthyl or anthryl, which can be substituted by C₁₋₁₈-alkyl, C₁₋₁₈-alkoxyl, carboxyl-C₁₋₁₇-alkyl or halogen.

3. Dental compounds according to claim 1 or 2, characterised in that as (d) they contain compounds of the general formula I, in which at least two of the radicals R¹, R², R³, particularly R² and R³ represent hydrogen.

4. Dental compounds according to claim 3, characterised in that as (d) they contain compounds of the general formula I, in which R¹ and Ar are the same and represent phenyl, C₁₋₆-alkyl-phenyl, C₁₋₆-alkoxylphenyl or carboxyl-C₁₋₆-alkyl.

5. Use of compounds of the general formula 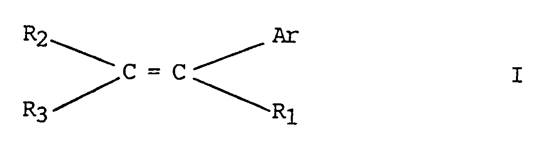 in which:
Ar represents an aryl or substituted aryl, which is substituted by straight-chain or branched C₁₋₁₈-alkyl, C₁₋₁₈-alkoxyl or carboxyl-C₁₋₁₇-alkyl or halogen,
R¹, R² and R³, which are the same or different, represent hydrogen, aryl or substituted aryl, which is substituted by straight-chain or branched C₁₋₁₈-alkyl, C₁₋₁₈-alkoxyl or carboxyl-C₁₋₁₇-alkyl or halogen, or represent straight-chain or branched C₁₋₁₈-alkyl, C₁₋₁₈-alkoxyl or C₁₋₁₇-alkoxycarbonyl, in which alkyl and alkoxyl can be substituted by halogen or aryl,
in which, if R¹ or R² represents aryl or substituted aryl, which is substituted by straight-chain or branched C₁₋₁₈-alkyl, C₁₋₁₈-alkoxyl or carboxyl-C₁₋₁₇-alkyl or halogen, or represents c₁₋₁₈-alkyl or C₁₋₁₈-alkoxyl, this radical can be linked with Ar by a single bond and
in which, if Ar represents phenyl, C₁₋₁₈-alkylphenyl, C₁₋₁₈-alkoxylphenyl, carboxyl-C₁₋₁₇-alkylphenyl or halophenyl, R² can also represent -O-, which is linked with the phenyl radical of Ar to a benzofuran,
and
in which at least one of the radicals R¹ to R³ represents H and at least one of the radicals R¹ to R³ represents aryl or aryl substituted by straight-chain or branched C₁₋₁₈-alkyl, C₁₋₁₈-alkoxyl or carboxyl-C₁₋₁₇-alkyl or halogen,
in the production of dental compounds, which contain bifunctional acrylic-acid and/or methacrylic-acid esters.

## Revendications

1. Compositions dentaires contenant :
(a) 10 à 99,999 % en poids d'un ester au moins bifonctionnel de l'acide acrylique et/ou méthacrylique,
(b) 0,001 à 5 % en poids d'un système d'initiateur qui peut provoquer la polymérisation radicalaire, et
(c) 0 à 89,999 % en poids de charges, d'auxiliaires de thixotropie et d'autres auxiliaires,
par rapport au poids total de (a) + (b) + (c),
caractérisées en ce qu'elles contiennent en outre :
(d) 0,001 à 10 % en poids, par rapport à (a), d'un composé répondant à la formule générale : 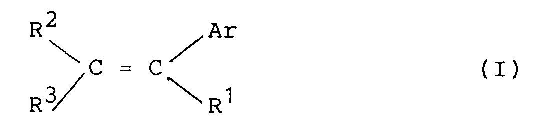 dans laquelle :
Ar signifie un reste aryle ou aryle substitué, qui est substitué par un alkyle en C₁₋₁₈, alcoxyle en C₁₋₁₈ ou carboxyl(alkyle en C₁₋₁₇), à chaîne droite ou ramifiée, ou par un atome d'halogène,
R¹, R² et R³, qui sont identiques ou différents, signifient un atome d'hydrogène, un reste aryle ou aryle substitué, qui est substitué par un alkyle en C₁₋₁₈, alcoxyle en C₁₋₁₈ ou carboxyl(alkyle en C₁₋₁₇) à chaîne droite ou ramifiée, ou par un atome d'halogène, un alkyle en C₁₋₁₈, alcoxyle en C₁₋₁₈ ou (alcoxy en C₁₋₁₇)carbonyle, à chaîne droite ou ramifiée, les restes alkyle et alcoxyle pouvant être substitués par un halogène ou un aryle,
où, quand R¹ ou R² signifie un reste aryle ou aryle substitué, qui est substitué par un alkyle en C₁₋₁₈, alcoxyle en C₁₋₁₈ ou carboxyl(alkyle en C₁₋₁₇), à chaîne droite ou ramifiée, ou par un atome d'halogène, alkyle en C₁₋₁₈ ou alcoxyle en C₁₋₁₈, ce reste peut être enchaîné à Ar par une liaison simple, et
où, quand Ar signifie un reste phényle, (alkyle en C₁₋₁₈)phényle, (alcoxyle en C₁₋₁₈)phényle, carboxyl(alkyle en C₁₋₁₇)phényle ou halogénophényle, R² peut aussi signifier -O-, qui est condensé avec Ar en un benzofuranne, et
où au moins un des reste R¹ à R³ signifie H, et au moins un des restes R¹ à R³ signifie un reste aryle ou aryle substitué par un alkyle en C₁₋₁₈, alcoxyle en C₁₋₁₈ ou carboxyl(alkyle en C₁₋₁₇), à chaîne droite ou ramifiée, ou par un halogène.

2. Compositions dentaires selon la revendication 1, caractérisées en ce qu elles contiennent, comme constituant (d), des composés de formule générale I, dans laquelle Ar signifie un phényle, naphtyle ou anthryle, qui peuvent être substitués par un alkyle en C₁₋₁₈, alcoxyle en C₁₋₁₈, carboxyl(alkyle en C₁₋₁₇) ou par un halogène.

3. Compositions dentaires selon la revendication 1 ou 2, caractérisées en ce qu'elles contiennent, comme constituant (d), des composés de formule générale I, dans laquelle au moins deux des restes R¹, R², R³, surtout R² et R³, signifient l'atome hydrogène.

4. Compositions dentaires selon la revendication 3, caractérisées en ce qu'elles contiennent, comme constituant (d), des composés de formule générale I dans lesquels R¹ et Ar sont identiques et ont la signification d'un phényle, (alkyle en C₁-C₆)phényle, (alcoxyle en C₁-C₆)phényle ou carboxyl(alkyle en C₁₋₆)phényle.

5. Utilisation de composés répondant à la formule générale : 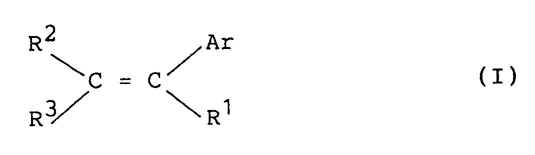 dans laquelle :
Ar signifie un reste aryle ou aryle substitué, qui est substitué par un alkyle en C₁₋₁₈, alcoxyle en C₁₋₁₈ ou carboxyl(alkyle en C₁₋₁₇), à chaîne droite ou ramifiée, ou par un atome d'halogène,
R¹, R² et R³, qui sont identiques ou différents, signifient un atome d'hydrogène, un reste aryle ou aryle substitué, qui est substitué par un alkyle en C₁₋₁₈, alcoxyle en C₁₋₁₈ ou carboxyl(alkyle en C₁₋₁₇) à chaîne droite ou ramifiée, ou par un atome d'halogène, un alkyle en C₁₋₁₈, alcoxyle en C₁₋₁₈ ou (alcoxy en C₁₋₁₇)carbonyle, à chaîne droite ou ramifiée, les restes alkyle et alcoxyle pouvant être substitués par un halogène ou un aryle,
où, quand R¹ ou R² signifie un reste aryle ou aryle substitué, qui est substitué par un alkyle en C₁₋₁₈, alcoxyle en C₁₋₁₈ ou carboxyl(alkyle en C₁₋₁₇), à chaîne droite ou ramifiée, ou par un atome d'halogène, alkyle en C₁₋₁₈ ou alcoxyle en C₁₋₁₈, ce reste peut être enchaîné à Ar par une liaison simple, et
où, quand Ar signifie un reste phényle, (alkyle en C₁₋₁₈)phényle, (alcoxyle en C₁₋₁₈)phényle, carboxyl(alkyle en C₁₋₁₇)phényle ou halogénophényle, R² peut aussi signifier -O-, qui est condense avec Ar en un benzofuranne, et
où au moins un des reste R¹ à R³ signifie H, et au moins un des restes R¹ à R³ signifie un reste aryle ou aryle substitué par un alkyle en C₁₋₁₈, alcoxyle en C₁₋₁₈ ou carboxyl(alkyle en C₁₋₁₇), à chaîne droite ou ramifiée, ou par un halogène,
pour la préparation de compositions dentaires qui contiennent des esters bifonctionnels de l'acide acrylique et/ou méthacrylique.
